Europäisches Patentamt

(19) European Patent Office

Office européen des brevets

(11) Publication number: **0 339 824**

**A1**

## (12) EUROPEAN PATENT APPLICATION

(21) Application number: **89303604.6**

(22) Date of filing: **12.04.89**

(51) Int. Cl.⁴: **C12M 1/00 , C12M 3/00 , B01L 7/02**

A request for correction of the drawings and description has been filed pursuant to Rule 88 EPC. A decision on the request will be taken during the proceedings before the Examining Division (Guidelines for Examination in the EPO, A-V, 2.2).

(30) Priority: **29.04.88 US 187815**

(43) Date of publication of application:
**02.11.89 Bulletin 89/44**

(84) Designated Contracting States:
**DE FR GB**

(71) Applicant: **BARNSTEAD THERMOLYNE CORPORATION**
**2555 Kerper Boulevard**
**Dubuque Iowa 52001(US)**

(72) Inventor: **Mahe, Stanley Raynold**
**2070 Key Largo**
**Dubuque Iowa 52001(US)**
Inventor: **Biver, William Ralph**
**1320 Oeth Court**
**Dubuque Iowa 52001(US)**

(74) Representative: **Oliver, Roy Edward et al**
**Pollak Mercer & Tench Eastcheap House**
**Central Approach**
**Letchworth Hertfordshire SG6 3DS(GB)**

(54) **Incubator.**

㉗ An apparatus (10) for incubating and/or incubating/shaking biological test specimens (145) has a detachable autoclavable chamber (12) provided with a support plate (104) and a diffuser plate (110) for directing a controlled flow of heated air to the biological test specimen; a transverse blower (40) produces a uniform air flow within the incubating chamber.

FIG 5

The present invention relates to incubators and incubator shakers, such as are used for maintaining cells, microorganisms and other biological test samples in a controlled environment.

A problem with prior art incubators and incubator shakers is their lack of ability to provide a uniform and tightly-controlled temperature within the incubating chamber. In certain laboratory tests, for example such as the enzyme immunoassay test for the AIDS virus, it is necessary to keep a set temperature within the chamber, generally in the range from 37° to 56°C. It is important that the temperature should not deviate more than ± 0.5°C. In this type of test, it is extremely important that the temperature throughout the incubator chamber should be substantially uniform. It is also important that the operating temperature should be reached in a quick, uniform and efficient manner. Typically, such tests have been conducted in a waterbath incubation device. However, waterbaths have the disadvantage of cross-contamination and an exposure risk to the technologist performing the test. Substitutes for waterbaths have been tried, such as aerobic incubators, but these do not offer uniform rapid heatup as compared to a waterbath. Another problem associated with prior art incubators is difficulty in cleaning and disinfecting the incubating chamber. For example, when a positive test result for the AIDS virus is obtained, it is important for the chamber to be thoroughly disinfected and/or sterilized. Prior art devices are limited to hand cleaning for this purpose. Another important matter in carrying out tests for contagious diseases is that the test specimens should not contaminate other test specimens. It is therefore important for the air flow within the incubating chamber to be carefully controlled, so as to minimize any potential cross-contamination.

This invention provides an improved incubator and incubator shaker, wherein a very uniform and constant temperature can be maintained, while minimizing or eliminating the problems of the prior art.

According to one aspect of this invention, an apparatus for incubating a biological test specimen is characterised by:

(a) a base section having drive means for providing rotating motion;

(b) a removable autoclavable incubating chamber having a shaker plate mounted therein for supporting a container containing the test specimen, connecting means for connecting the shaker plate to the drive means and blower means for producing an air flow within the incubator chamber;

(c) coupling means for connecting the drive means to the connecting means; and

(d) blower drive means, for driving the blower means, detachably mounted on the incubator chamber.

In one aspect of the present invention, an apparatus for incubating a biological test specimen is provided, which includes a base support section, a removable incubating chamber and a blower drive means detachably mounted in or on the incubating chamber.

In accordance with another aspect of the present invention, an apparatus for incubating a biological test sample comprises a main incubating chamber, in which a support plate is provided, for supporting test specimens. The support plate has a plurality of openings, one associated with each test specimen. A diffuser plate is provided between the support plate and the bottom wall of the chamber for providing a pressure staging area between the support plate and the diffuser plate and an air receiving area between the diffuser plate and the bottom of the incubating chamber.

In order that the invention may be fully understood, an embodiment of the apparatus is described below, by way of example only, in conjunction with the accompanying drawings, wherein:

Figure 1 is a front perspective view of an apparatus made in accordance with the present invention;

Figure 1A is a back perspective view of the apparatus of Figure 1;

Figure 2 is an exploded perspective view of the parts of the apparatus of Figure 1;

Figure 2A is a fragmentary view of the bottom of an incubating chamber assembly in the apparatus of Figure 1;

Figure 3 is a front elevational view of the apparatus of Figure 1;

Figure 4 is a side elevational view of the apparatus of Figure 1;

Figure 5 is a cross-sectional view of the incubating chamber of the apparatus taken along the line 5-5 of Figure 2;

Figure 6 is a cross-sectional view of the incubating chamber, showing the diffuser plate mounted in the incubator chamber, taken along line 6-6 of Figure 5;

Figure 7 is a cross-sectional view of the incubating chamber, showing the shaker plate, taken along the line 7-7 of Figure 5;

Figure 8 is a cross-sectional view of the incubating chamber of Figure 5, taken along line 8-8 and showing the air receiving area;

Figure 8A is an enlarged view of the heating element of the heater shown in the circle in Figure 8;

Figure 9 is a side cross-sectional view of the incubating chamber of Figure 5 taken along the line 9-9;

Figure 10 is a side cross-sectional view of the incubating chamber of Figure 5 taken along the line 10-10;

Figure 11 is a back elevational view of the apparatus of Figure 1;

Figure 12 is an enlarged cross-sectional partial view; showing the connection of the blower drive assembly to the incubating chamber; and

Figure 13 is a front plan view of the blower drive assembly as taken along the line 13-13 of Figure 11.

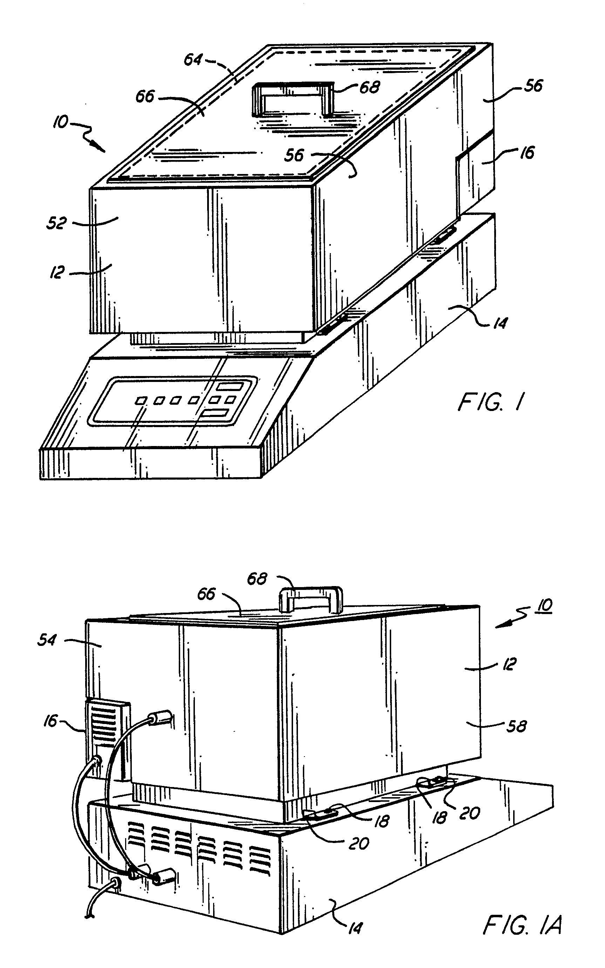

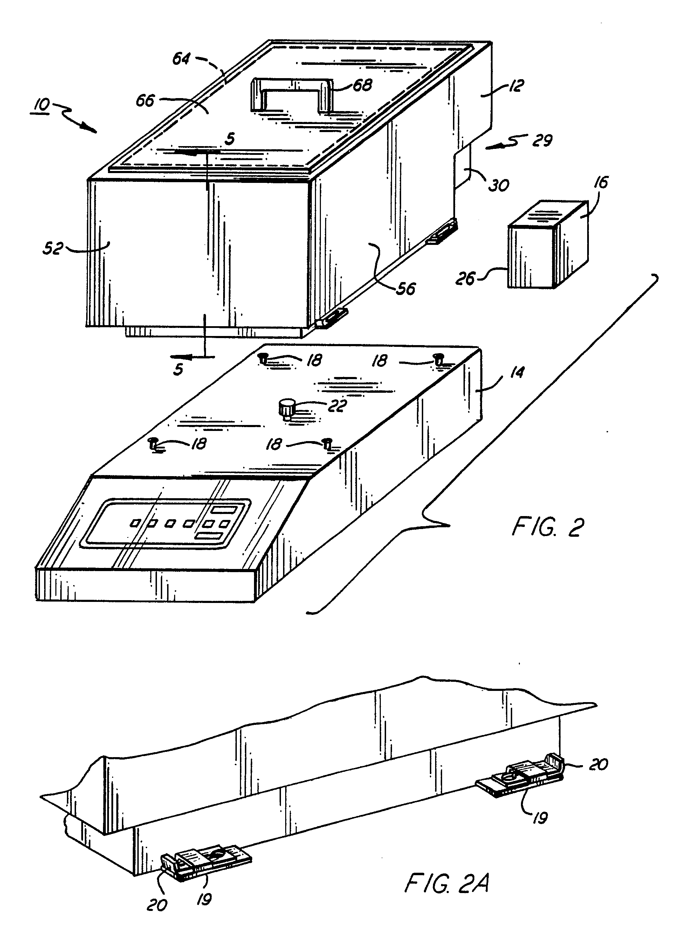

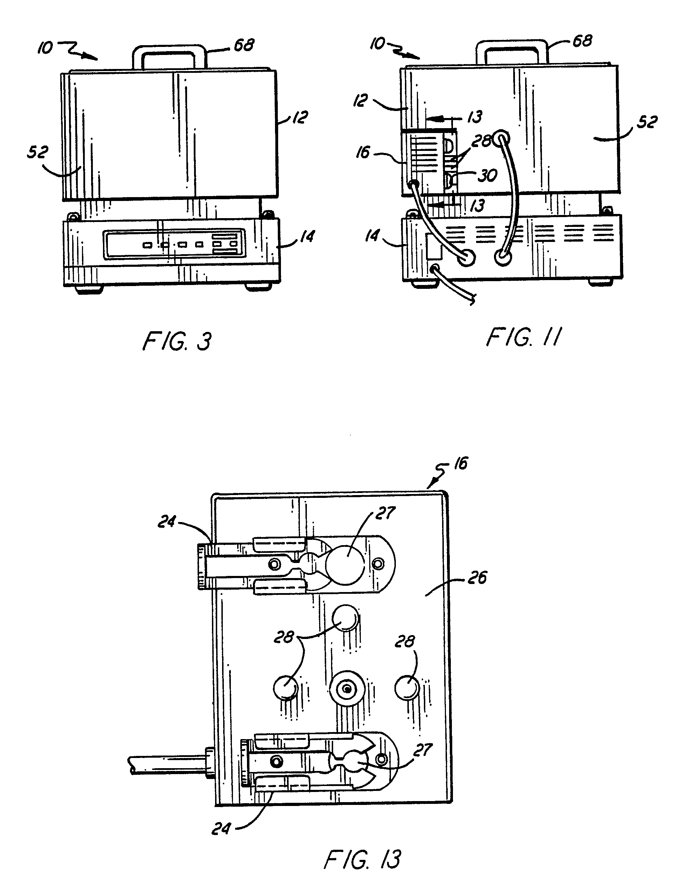

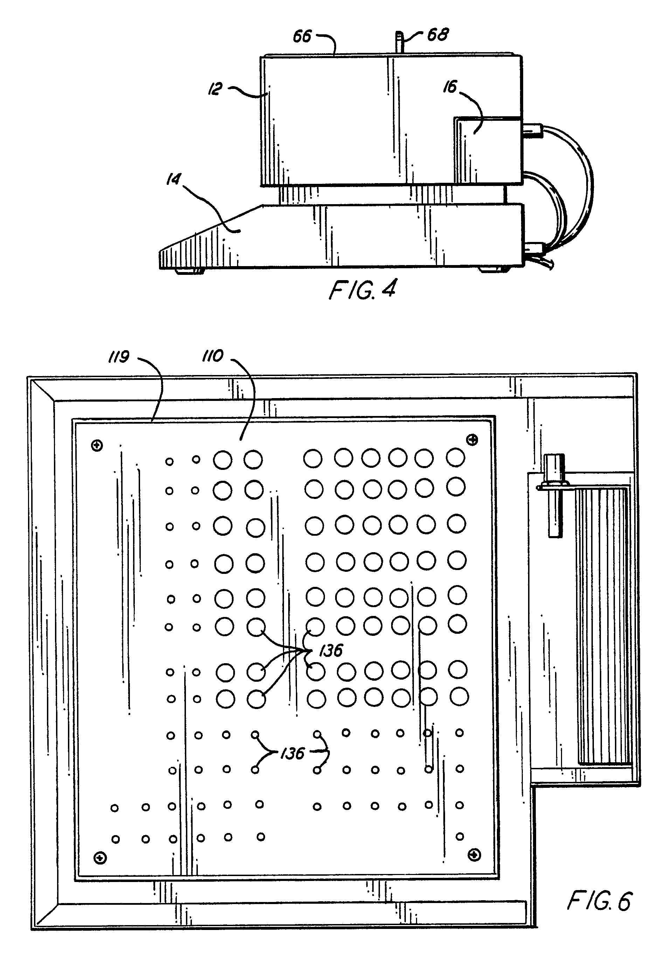

Referring to Figures 1-4, these show an incubation apparatus 10 in accordance with the present invention. The apparatus 10 may take the form of an incubator or an incubator shaker. The apparatus 10 comprises three separate components, namely a base section 14, an incubating chamber assembly 12 which is detachably mounted on the base section 14 and a blower drive assembly 16 detachably mounted on the incubating chamber assembly 12. The incubating chamber assembly 12 has a plurality of mounting pins 18 (Figs. 1A and 2) for location within receiving latches 20 (Figure 2A) mounted on mounting flanges 19 provided on the incubator assembly 12. The mounting pins 18 and the corresponding latches 20 are designed to secure the incubating chamber assembly 12 firmly on the base section 14. It is to be understood that various other means may be used for detachably mounting the incubator chamber assembly 12 on the base section 14.

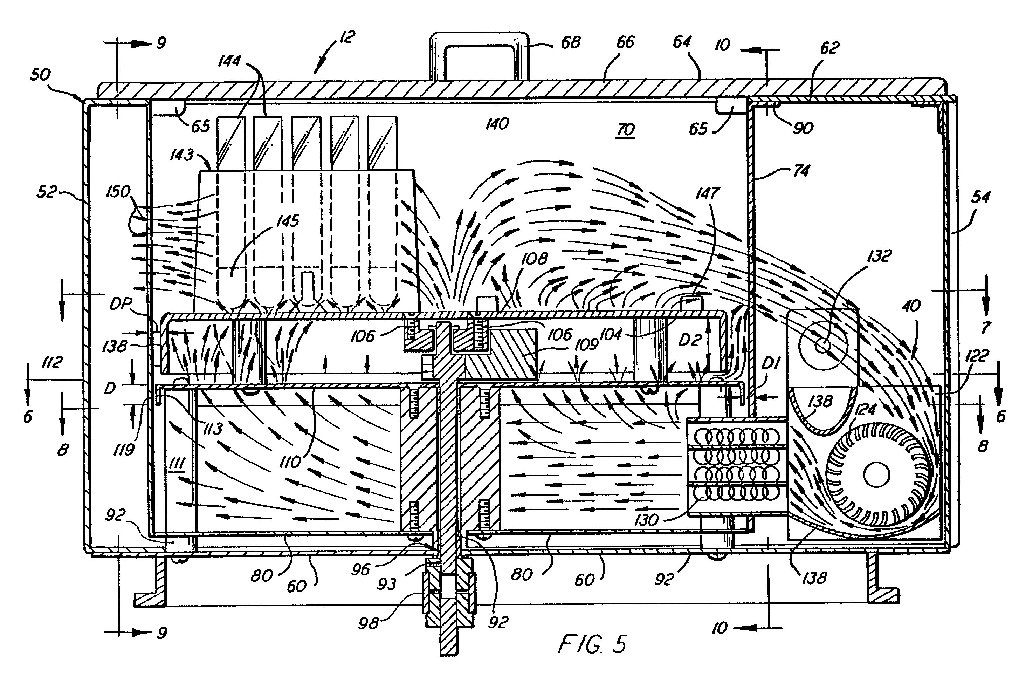

The base section 14 houses control means for determining the temperature of incubation and various other controlled functions, as is typically done in prior art devices. Appropriate sensors are connected by appropriate detachable cables between the base section 14 and the incubator chamber assembly 12. The base section 14 also houses an agitating motor, which has an output drive shaft 22 (Fig. 2) capable of connection to a drive shaft 92 in the incubating chamber assembly 12, as described in more detail below (see Fig. 5).

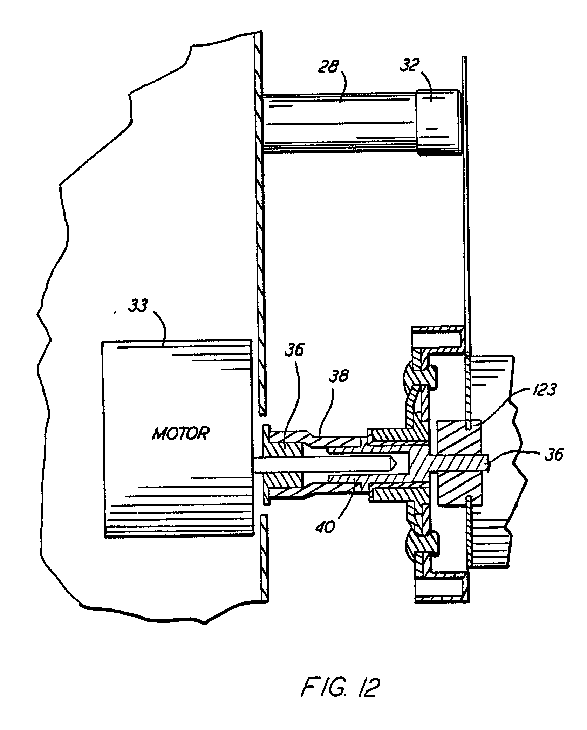

Referring to Figs. 11, 12 and 13, the blower drive assembly 16 is shown in greater detail. A pair of slidable mounting clips 24 are secured to the mounting face 26 of the blower drive assembly 16. These mounting clips 24 are designed to engage a pair of aligned mounting pins 25 (Figs. 11 and 13) secured to the chamber assembly 12. The pins 25 enter an opening 27 in the corresponding clip 24 and, when the clips 24 are pushed in, they engage and clamp the pins 25, so as to secure the blower drive assembly 16 firmly to the chamber assembly 12. Likewise, when the clips 24 are pulled out, they release the pins 25, to allow removal of the blower drive assembly 16. In the present invention, the chamber assembly 12 and the blower drive assembly 16 are shaped such that, when attached to one another, they form a generally smooth outer configuration. This is accomplished by providing the chamber assembly 12 with a recess area 29 (Fig. 2) which has a configuration which corresponds to the outer configuration of the blower drive assembly 16. It is, of course, understood that the mounting means for securing the blower drive assembly 16 on the incubator chamber assembly 12 may take any desired form and the outer configuration need not be smooth.

Fig. 12 shows, on an enlarged scale and in section, how the blower drive assembly 16 is mounted on the incubator chamber assembly 12. The blower drive assembly 16 has three mounting spacers 28 (see Fig. 13) which maintain the blower drive assembly 16 at a predetermined distance from a mounting face 30 (Fig. 2) in the recess area 29 in the incubating chamber assembly 12. Preferably, as shown, these mounting spacers 28 are provided with rubber cushions 32 for absorbing vibrations between the blower drive assembly 16 and the incubating chamber assembly 12.

The blower drive assembly 16 has a blower drive motor 33 (Fig. 13) mounted therein which turns a drive bushing 36. A flexible cylindrical drive coupling 38 is secured to the bushing 36, by simply pressing one end over the drive bushing 36. The other end of the flexible cylindrical drive coupling 38 is connected to the drive shaft 41 of the blower assembly 40 mounted within the incubating chamber assembly 12. The flexible coupling 38 provides a simple but reliable means for compensating for any misalignment between the drive shaft 41 and the drive bushing 36. This avoids the necessity of providing an expensive alignment feature.

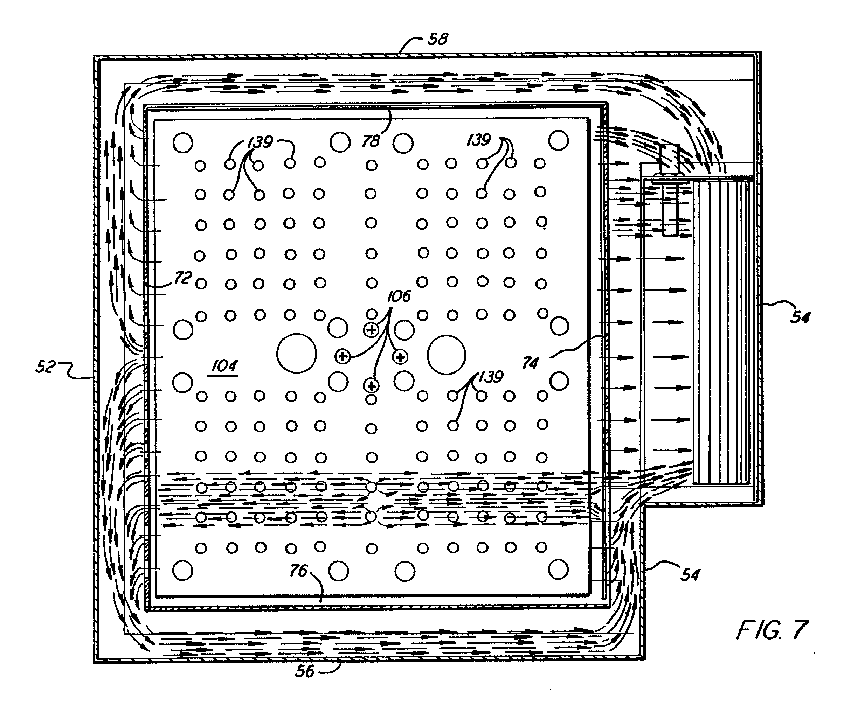

Referring to Figures 5 and 7, these show cross-sectional views of the incubator chamber assembly 12. The incubator chamber assembly 12 comprises a housing 50 having a front wall 52, a back wall 54, a right side wall 56, a left side wall 58, a bottom wall 60 and a top wall 62. The top wall 62 is provided with an access opening 64, to allow biological test specimens to be placed in or removed from the housing 50 of the incubator assembly 12. A removable top cover 66 is provided for closing the access opening 64. This cover 66 is provided with four spacers 65 on its bottom surface, for aligning the top cover 66 with the opening 64. The cover 66 is also provided with a handle 68 for lifting it off the access opening 64. Within the housing 50, an inner main chamber 70 is provided, which comprises a front wall 72, a back wall 74, a right side wall 76 and a left side wall 78 and the chamber 70 also has a bottom wall 80, which interconnects the front, back, right side, and left side walls 72, 74, 76, 78. Each of these walls 72, 74, 76 and 78 is provided with a flange 90 at the top, adjacent the top wall 62 of the housing 50. Preferably, a silicone rubber layer is placed between the flanges 90 and the top wall 62. The bottom wall 80 of the chamber 70 is supported by spacers 92 (Fig. 5) which are secured to the bottom wall 60 of the housing 50.

An eccentric or offset shaft 92 (mentioned above in relation to Fig. 2) is mounted substantially centrally in the chamber 70, so as to rotate about its axis. The offset shaft 92 has a lower end 93, which extends through an opening 96 in the bottom wall 60, and engages a coupling 98, connected to the output drive shaft with a counterweight 109 to compensate for the off-centre rotation. A bearing 103 is secured to the upper end 102 of the shaft 92 and is disposed in a bearing holder (not shown) attached to a support plate 104. In the particular embodiment illustrated, the bearing holder is attached to the shaft 92 by a retaining clip (not shown) and the support plate 104 is secured to the bearing holder with four screws 106, which pass through openings 108 in the support plate 104. While screws are shown, for securing the support plate 104 to the shaft 92, any other desired means may be used for the purpose. The plate 104 has an outer peripheral edge 117, which is designed to be as close as possible to the walls 72, 74, 76 and 78. However, due to its orbital rotation, the plate 104 will vary in its distance from the walls 72, 74, 76 and 78. Preferably, the plate 104 is designed such that its peripheral edge 117, at its closest point to the walls 72, 74, 76 and 78 is spaced from them by a distance DP of about 1/16 inch (1.6 mm).

Referring to Figs. 5 and 6, a diffuser plate 110 is provided within the main chamber 70 and extends substantially over it cross-sectional area, as shown, so as to provide a receiving area 115 between the bottom wall 80 and the diffuser plate 110. The diffuser plate 110 may be secured to the housing 50 in any desired manner. In the embodiment shown, for instance, the diffuser plate 110 is secured (Fig. 5) to spacers by means of screws 112 and these spacers 111 are secured to further spacers 192 which are secured to the bottom wall 80. The diffuser plate 110 has an outer peripheral skirt 114 which extends downwards by a distance D. It has been found that the downward extent D of the skirt 113 should be at least 1/4 inch (6 mm) and, preferably, at least 1 inch (25 mm). The diffuser plate 110 is provided with a plurality of openings 136, which are sized and located so as to provide a substantially uniform pressure area between the diffuser plate (110) and the underside of the support plate 104. The support plate 104 has a downwardly-extending skirt 138, which extends over a distance, D2, which is preferably at least 1/2 inch (12 mm). In the particular embodiment shown, the downward extent D2 of the skirt 138 is about 3/4 inch (19 mm).

The particular size and location of the openings 136 is advantageously arrived at, as was done in developing the apparatus of the invention, by trying various combinations of size and location until the desired pressure distribution is obtained. As shown in Figure 6, the upper right portion of the diffuser plate 110 is located as close as possible to the walls 72, 74, 76 and 78. In the embodiment shown, the edge 119 is spaced from the walls 72 to 78 by a distance, D1, of about 1/16 inch (1.6 mm).

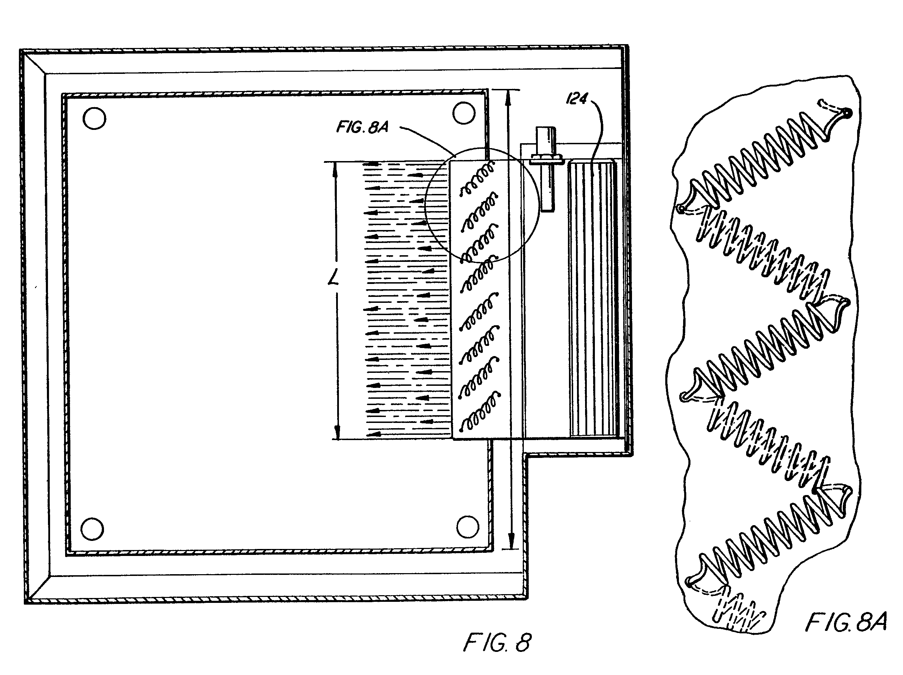

A blower assembly 40 (Fig. 5) is provided within the incubating chamber assembly 12 and is located such that its output is in direct communication with the receiving area 115 between the diffuser plate 110 and the bottom wall 80 of the inner chamber 70. The blower assembly 40 includes a housing 122, which contains a squirrel-cage impeller 124 mounted on side rails 126 of the housing 122 by bushings 123. The impeller 124 is designed to receive air through openings in the top of the housing 122 and to direct it out from a side wall 128 through an outlet port 129 into the receiving area 115 of the inner main chamber 70. Placed at the exit from the blower assembly 40 is a heating means, which heats the air entering the inner chamber 70 to the preselected temperature. In the particular embodiment shown, the heating means comprise heating coils 130 arranged in a zig-zag pattern (see Figures 8 and 8A) which are made of an autoclavable material. The elements or coils 130 extend substantially across the entire length of the outlet port 129.

Baffles 131 (Fig. 5) are provided for appropriately directing the air flow from the impeller 124 into the inner chamber 70. The impeller 124 is designed to extend substantially across the width W of the chamber 70. The outlet port 129 has a length L which is at least 30% of the width W of the inner chamber 70. Preferably, the length L of the outlet port 129 is at least 50% of the width W of the chamber 70. In the particular embodiment shown, the length L is approximately 60% of W. This helps to ensure a more uniform airflow and pressure distribution within the receiving area 115. The heating elements 130 are controlled by a solid state temperature controller, as is well known in the art, in response to a temperature sensor 132 disposed on the top of the blower assembly 40. In the particular embodiment shown, the impeller 124 is designed to provide an air flow of approximately 36 cfm ($1m^3$/min). The air which enters the receiving area 115 is forced upwards through the openings 136 in the diffuser plate 110. The openings 136 having a size and are located so as to provide a substantial uniform pressure between the diffuser plate 110 and the bottom of the support plate 104. The skirt 138 helps to provide a staging area for the air passing through the diffuser plate 110, prior to entering the main incubation are 140 above the support plate 104.

The support plate 104 is provided with a plurality of openings 139 (Fig. 7), each opening 139 being designed to be associated with a biological test specimen. As can be seen in Fig. 5, two test tube racks 143 are provided, each having a plurality of test tubes 144 therein. Each test tube 144 has in it a biological test specimen 145, to be exposed to the incubation environment. The test tube racks 143 are positioned by means of a plurality of spacers 147, designed to mate with the test tube racks 143 so as to hold them firmly in position. Each test tube rack 143 has an appropriate matrix for holding a desired number of test tubes 144. The support plate 104 is designed such that one opening 139 is associated with each test tube and is located substantially below where the specimen is placed.

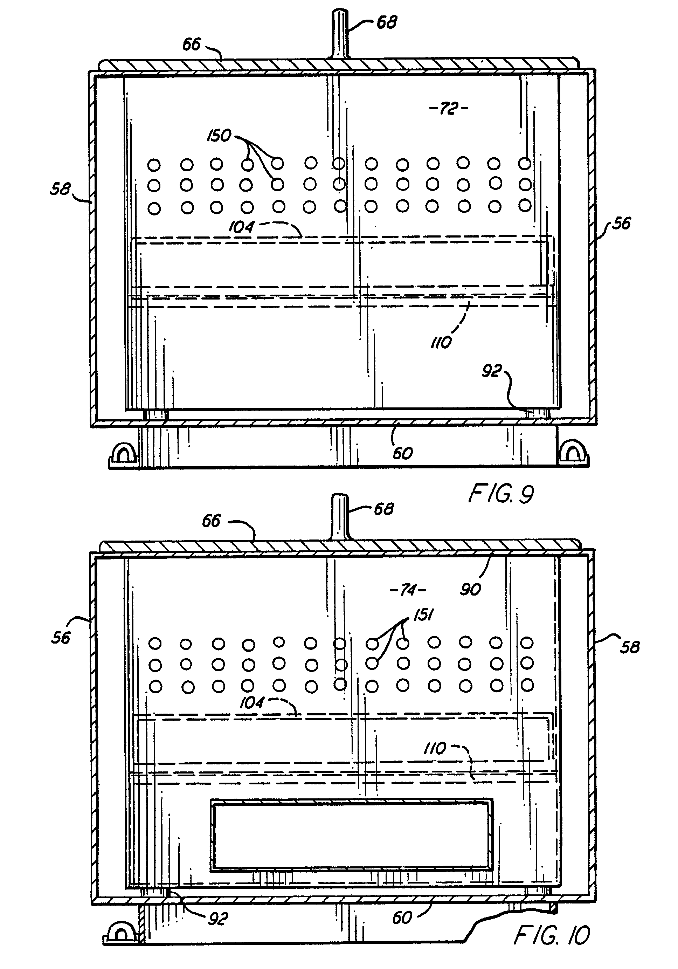

The front wall 72 of the chamber 70 (see Fig. 9) is provided with a plurality of openings 150, which are located above the support plate 104 and below the tops of the test tubes 144. Air is drawn through the openings 150 between the sides of the housing 50 and the inner chamber 70 to the blower assembly 40. The back wall (Fig. 10) is also provided with a plurality of openings 151, which are disposed above the support plate 104 and below the tops of the test tubes 144. It has been found that it is important to avoid air flow around the tops of the test tubes 144 (or other containers), as this can cause siphoning or cross-contamination between the biological test specimens in the test tubes 144.

Operation of the apparatus 10 according to the present invention takes place as follows. The top cover 66 is removed to allow access to the inside of the inner chamber 70. A plurality of test tube racks 143 (typically 2 or 4) are placed in the chamber 70, each test tube 144 having a biological specimen in it. The racks 143 are designed to mate with the support plate 104. After the racks 143 have been properly secured to the support plate 104, the top cover 66 is placed on the incubator/shaker. The appropriate test conditions are established in the apparatus, as is commonly done in the prior art. Thereafter, the heater and blower assemblies 130 and 40 are turned on, to provide circulation of heated air in the chamber 70. The arrows (Fig. 5) show how the air flow typically takes place. The sensor 132 monitors the temperature within the chamber 70 and so controls the amount of heat supplied by the heating elements.

Air flows into the receiving area 115 from the blower assembly 40 at a relatively high velocity. The openings 136 in the diffuser plate 110, located directly below each specimen, direct the air toward the support plate 104, in a manner such that the pressure above the diffuser plate 110 is substantially uniform. The air then flows around the bottom test tubes 144 and out of the front or back walls 72, 74 through the appropriate openings therein. As can be seen, the air passing out via the front wall 72 goes through side passages back to the blower assembly 40. This type of air movement provides for quick heating of the inner chamber 70 to its operating temperature and allows for efficient exchange of heat, to allow the sensor 132 to provide extremely precise temperature control.

The support plate 104 is designed for orbital movement, as is well known in the art of incubator shakers. Thus, at the appropriate time, the apparatus activates the drive motor 33 in the base section 14, causing the plate 104 to undergo its pre-designed shaking motion.

It has been found that an apparatus in accordance with the present invention can bring the temperature to the desired operating range within 6 to 10 minutes and can maintain it within 0.5°C uniformly throughout the entire chamber 70.

After the operating cycle has been completed, the test specimens are removed and the chamber 70 is appropriately cleaned down by an appropriate method. In certain instances, it is desirable for the incubating chamber to be thoroughly cleaned. Typically, in the prior art, this is accomplished by hand swabbing with a cotton swab, although it is generally difficult to get into the tight corners of the incubating chamber and surrounding areas. Thus, the present invention provides means whereby the incubating chamber assembly 12 may be easily and quickly removed from the base section 14 and the blower drive or motor assembly 16 can be readily removed from the incubator chamber assembly 12. The incubator chamber assembly 12 can then be placed directly in an autoclave or steam sterizilser for sterilization. since the incubating chamber assembly 12 can be thoroughly disinfected, this minimizes any chance of later contamination or a potential health hazard to users of the apparatus.

It is understood that various modifications can be made without the parting from the scope of the present invention. For example, the support plate may be designed to use test specimens other than test tubes. For example, the support plate can be designated to hold titrator plates.

## Claims

1. An apparatus (10) for incubating a biological test specimen (145), characterised by comprising:

(a) a base section (14) having drive means (33) for providing rotating motion;

(b) a removable autoclavable incubating chamber (12) having a shaker plate (104) mounted therein for supporting a container (144) containing the test specimen, connecting means (92,93) for connecting the shaker plate to the drive means and blower means (40) for producing an air flow within the incubator chamber;

(c) coupling means (98) for connecting the drive means to the connecting means; and

(d) blower drive means (16), for driving the blower means, detachably mounted on the incubator chamber.

2. An apparatus according to claim 1, wherein a flexible coupling means (98) is provided for connecting the blower drive means to the blower means.

3. An apparatus according to claim 2, wherein the flexible coupling means comprise a flexible sleeve (98) having one end securable to the drive means and another end secured to a shaft (41) connected to the blower means.

4. An apparatus according to any preceding claim, wherein the base section houses control means for operating and controlling the incubator chamber.

5. An apparatus according to any preceding claim, wherein the incubating chamber is disposed in a housing (50) having bottom (60), top (62) and side (52, 54, 56, 58) walls, a diffuser plate (110) spaced above the bottom surface of the main chamber and forming a receiving section for receiving the air flow from the blower means, a support plate (104) spaced above the diffuser plate in the main chamber which forms a secondary air staging area between the support plate and the diffuser plate, the diffuser plate having a plurality of openings (136) in such number, size and location as to provide a substantially uniform pressure within the secondary receiving area, and the support plate having a plurality of openings (139), each disposed so as to be associated with a biological test specimen.

6. An apparatus according to claim 5, wherein the main incubating chamber has peripheral walls (76, 78), a bottom wall (80) and a top wall, an outer chamber surrounding side and bottom walls of the main incubating chamber.

7. An apparatus according to claim 5 or 6, wherein the diffuser and support plates extend substantially over the cross-sectional area of the main incubating chamber and are spaced from the side wall of the main incubating chamber.

8. An apparatus according to claim 5, 6 or 7, wherein the support plate has an outer skirt (138) extending downwardly around substantially its entire outer periphery by a distance of at least 0.5 inch (12 mm).

9. An apparatus according to any of claims 5 to 8, wherein the diffuser plate has an outer skirt (113) extending downwardly around substantially its entire outer periphery by a distance of at least 1/4 inch (6 mm).

10. An apparatus according to any preceding claim, wherein the blower means extend across at least 50% of the width of the main incubating chamber.

11. An apparatus according to claim 10, wherein the blower means are arranged to provide an air flow at the bottom of the incubating chamber at right-angles thereto and are disposed substantially centrally of the incubating chamber.

12. An apparatus (10) for incubating a biological test specimen (145), characterised by a housing (50) having a main incubating chamber (70), blower means (40) for providing air within the incubating chamber, the blower means extending across at least 50% of the width of the incubating chamber, providing an air flow at right-angles to the incubating chamber at the bottom thereof and being disposed substantially centrally of the incubating chamber.

13. An apparatus (10) for incubating a biological sample (145), characterised by comprising a housing (50) having a main incubating chamber (70) having outer peripheral front, back and side walls (72-78), a bottom wall (80) and a top wall (62), an outer chamber (50) surrounding the side and bottom walls of the main incubating chamber, blower means (40) located within the outer chamber for blowing air into the bottom of the main incubating chamber, an air diffuser plate (110) disposed in the chamber (70) and spaced from its bottom surface, so as to form an area for receiving air from the blower means, and a support plate (104) disposed above the diffuser plate and forming an air staging area for the air flow so as to stabilize the pressure and provide an incubation test area above the support plate.

14. An apparatus according to claim 13, wherein the diffuser plate has a plurality of openings (136) in such number, size and location as to provide a substantially uniform pressure within the secondary receiving area, and the support plate has a plurality of openings (139), each disposed so as to be associated with a respective biological test specimen.

15. An apparatus according to any preceding claim, wherein a heater means (139, 131) is provided directly after the blower means and extends across substantially the entire length of the blower means.

FIG. 1

FIG. 1A

FIG. 2

FIG. 2A

*FIG. 3*

*FIG. 11*

*FIG. 13*

FIG. 4

FIG. 6

FIG. 5

FIG. 7

EP 0 339 824 A1

FIG. 8

FIG. 8A

124

EP 0 339 824 A1

FIG. 9

FIG. 10

FIG. 12

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 572 427 (G.D. SELFRIDGE et al.) * Column 3, lines 50-61; column 4, line 48 - column 5, line 64; column 8, lines 52-58; claim 1; figures 2,4 * | 1,4-9, 13,14 | C 12 M 1/00 C 12 M 3/00 B 01 L 7/02 |
| A | US-A-3 634 651 (M.E. SIEGEL et al.) * Column 4, lines 28-49; column 4, line 69 - column 5, line 2; column 5, lines 40-60; claims 1,4,7,8,10; figures 4,5 * | 1,4-7, 10-15 | |
| A | GB-A-1 112 919 (I. ROLOVICH) * Page 2, line 106 - page 3, line 40; claims 1,4; figure 1 * | 1 | |
| A | EP-A-0 165 172 (INSERM) * Page 5, line 11 - page 6, line 23; page 11, line 33 - page 12, line 30; claim 1; figures 1,2 * | 1 | |
| A | DE-U-8 601 344 (F. DREBENSTEDT) * Page 3, lines 1-8; page 4, lines 15-23; page 5, lines 1-4; claims 1,6; figure 1 * | 1 | |

TECHNICAL FIELDS SEARCHED (Int. Cl.4)

C 12 M
B 01 L

The present search report has been drawn up for all claims

| Place of search | Date of completion of the search | Examiner |
|---|---|---|
| THE HAGUE | 09-08-1989 | GROENENDIJK M.S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document